# FASCICULE DE BREVET EUROPEEN

(11) **EP 1 140 842 B1**
(45) Date de publication et mention de la délivrance du brevet: **02.11.2005**
(21) Numéro de dépôt: 99973487.4
(22) Date de dépôt: 01.12.1999
(51) Int. Cl.: C07D 211/88, C07C 255/20, C07C 255/22

(54) **ESTERS D'ALKYLE DE L'ACIDE 3-(3,4-DIHALOGENOPHENYL)-2,6-DIOXOPIPERIDINE-3-PROPIONIQUE UTILES COMME INTERMEDIAIRE**
ESTER VON 3-(3,4-DIHALOGENOPHENYL)-2,6-DIOXOPIPERIDINE-3-PROPIONSÄURE ALS ZWISCHENPRODUKTE
ALKYL ESTERS OF 3-(3,4-DIHALOGENOPHENYL)-2,6-DIOXOPIPERIDINE-3-PROPIONIC ACID USEFUL AS INTERMEDIATES

(30) Priorité: 18.12.1998 FR 9816087
(43) Date de publication de la demande: 10.10.2001
(73) Titulaire: Sanofi-Aventis, 75013 Paris (FR)
(72) Inventeur: CASTRO, Bertrand, F-34130 Saint Aunes (FR); DORMOY, Jean-Robert, F-04020 Sisteron (FR); RABION, Alain, F-64000 Pau (FR)
(74) Mandataire: Thouret-Lemaitre, Elisabeth
(86) Numéro de dépôt international: PCT/FR1999/002970
(87) Numéro de publication internationale: WO 2000/037445

(56) Documents cités:
- EP-A- 0 673 928
- WO-A-97/32852
- WO-A-98/05640
- WO-A-99/01451

## Description

La présente invention a pour objet un ester d'alkyle inférieur d'acide 3-(3,4-dihalogenophényl)-2,6-dioxopipéridine-3-propionique de configuration S.

L'invention concerne également un procédé de préparation de ce composé et l'utilisation dudit composé pour préparer l'acide correspondant.

L'acide 3-(3,4-dichlorophényl)-2,6-dioxopipéridine-3-propionique est décrit dans la demande de brevet WO 97/32852. Selon cette demande de brevet, l'acide 3-(3,4-dichlorophényl-2,6-dioxopipéridine-3-propionique peut être réduit, par exemple par le borane pour donner le 3-(3,4-dichlorophényl)-3-(3-hydroxypropyl)pipéridine. Ce dernier composé, décrit dans la demande de brevet EP-A-673928, est un intermédiaire utile dans la préparation de l'osanétant. L'osanétant est un antagoniste spécifique des récepteurs NK₃, décrit, en particulier, par X. Emonds-Alt dans Life Sci., 1995, 56 (1), 27-32.

On a maintenant trouvé un composé nouveau de configuration S répondant à la formule : 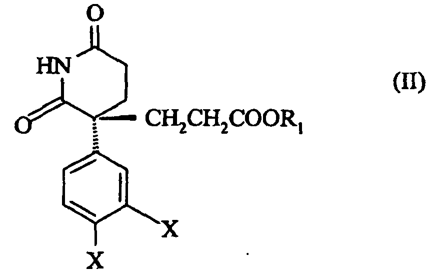 dans laquelle :
- X représente un halogène, préférentiellement un atome de chlore ou de fluor ;
- R₁ représente un alkyle linéaire en C₁-C₄, préférentiellement un méthyle.

La présente invention est tout particulièrement relative au composé de formule (II) dans laquelle X = Cl et R₁ = CH₃.

Selon le procédé de l'invention, on peut préparer l'isomère optique de formule (II) sous forme optiquement pure par un procédé caractérisé en ce qu'on effectue une hydrolyse enzymatique énantiosélective du composé de formule :

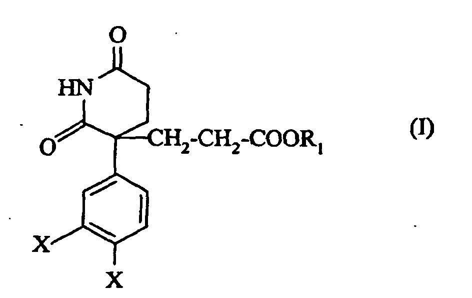

Ainsi, on hydrolyse le composé racémique de formule (I) par une enzyme choisie parmi les lipases, les protéases ou les estérases, les lipases ou les estérases étant préférées.

A titre d'exemples non limitatifs, on peut citer les lipases ou estérases de *Candida Cylindracea, Candida Rugosa, Pseudomonas Flurorescens, Humilica Lanuginosa, Candida Lipolytica,* α-chymotripsin, ou l'estérase de foie de porc.

On préfère l'estérase ou la lipase de *Candida Rugosa,* ou de *Candida Cylindracea,* séparément ou en mélange.

Ces enzymes sont utilisées sous forme purifiée ou sous forme d'extraits bruts. Les enzymes peuvent être ou non fixées sur un support.

La réaction d'hydrolyse est effectuée selon le schéma réactionnel suivant :

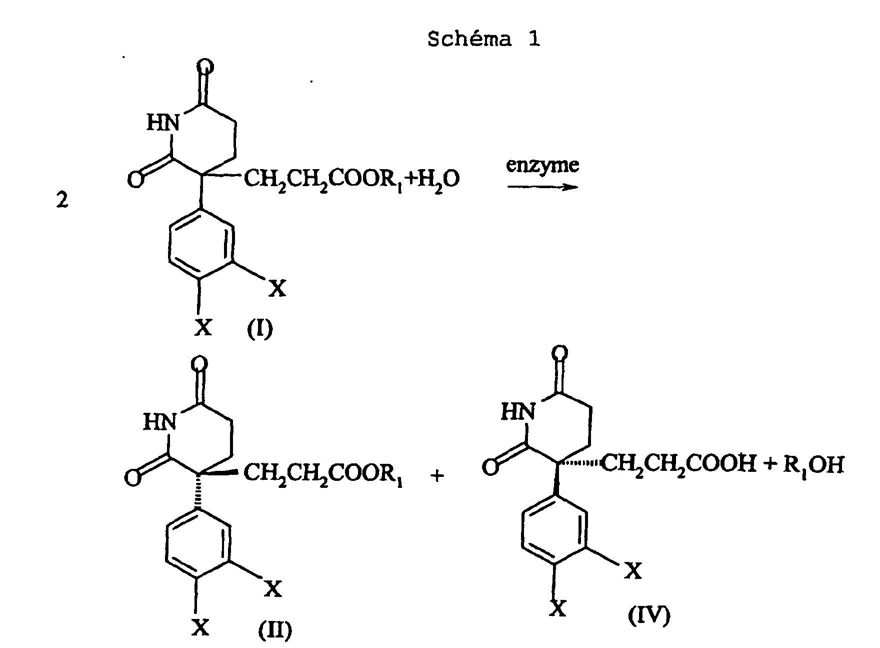

L'hydrolyse enzymatique selon l'invention est effectuée dans un milieu contenant de l'eau et un solvant organique. Le solvant organique peut être apolaire ou moyennement polaire, tel qu'un éther en C₁-C₁₀, un alcane en C₁-C₁₀, un alcool tertiaire en C₁-C₁₀, une cétone en C₁-C₁₀, un sulfoxide en C₁-C₁₀, le furane, ou, dans certains cas, un solvant chloré tel que le dichlorométhane, ces solvants étant utilisés purs ou en mélange.

De façon préférentielle, on utilise un éther aliphatique en C₁-C₁₀, tout particulièrement le méthyl tert-butyléther.

L'eau nécessaire à l'hydrolyse peut être solubilisée dans le milieu réactionnel par un co-solvant polaire ou bien, de façon préférentielle, l'eau constitue une phase à part, la réaction de l'hydrolyse étant alors réalisée en milieu biphasique.

Ainsi, on préfère tout particulièrement effectuer la réaction dans un milieu biphasique constitué par méthyl *tert*-butyléther (MTBE) et eau. Le rapport MTBE/eau peut varier de 1/99 à99/1, on préfère un rapport de l'ordre de 40/60 à 50/50, tout particulièrement 44/56.

L'eau utilisée peut être tamponnée ou non et son pH peut varier de 4 à 10 environ, préférentiellement on utilise de l'eau dont le pH est de l'ordre de 5 à 8.

La concentration du milieu réactionnel en diester peut varier dans les proportions 1 à 500 g/l et préférentiellement de 1 à 150 g/l tandis que la quantité d'enzyme varie dans des proportions de 0.0001 à 150 g/l et de préférence de 1 à 50 g/l.

La température de la réaction d'hydrolyse enzymatique peut varier entre 0°C et +50°C et de préférence entre +16°C et +35°C.

La durée de la réaction est comprise entre 3 heures et 36 heures, généralement voisine de 10 heures.

L'ester chiral de formule (II) est isolé par extraction après avoir précipité puis filtré l'enzyme utilisée.

Selon la présente invention, on peut également préparer un composé de formule (II) par un procédé consistant à effectuer une cyclisation du composé de formule : 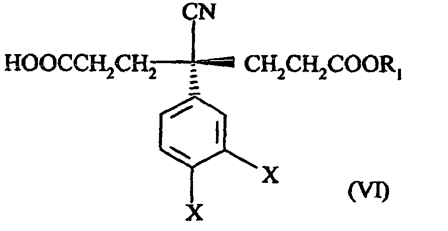 dans laquelle R₁ et X sont tels que définis ci-dessus pour (II).

La cyclisation du composé de formule (VI) est réalisée en présence d'un catalyseur.

On peut effectuer la cyclisation en présence d'un catalyseur tel qu'un anhydride d'acide, par exemple l'anhydride acétique, l'anhydride phosphorique, l'anhydride triflique, l'anhydride trifluoroacétique ou l'anhydride méthanesulfonique ou un acide tel que l'acide méthanesulfonique ou l'acide triflique, ou un mélange d'un anhydride d'acide et d'un acide.

Comme catalyseur, on préfère utiliser l'anhydride méthanesulfonique et l'acide méthanesulfonique ou l'anhydride triflique et l'acide triflique.

La réaction de cyclisation est effectuée à une température comprise entre 20°C et 130°C, de préférence entre 70°C et 120°C.

Pour effectuer la cyclisation du composé de formule (VI), on utilise préférentiellement la cyclisation catalytique qui permet de conserver la pureté optique.

Le composé de formule (II) est isolé du milieu par extraction en utilisant les conditions connues de l'homme de l'art.

Le composé de formule (VI) est obtenu par un procédé consistant à traiter par une enzyme un composé de formule : 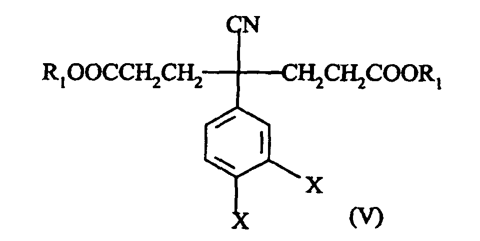 dans laquelle R₁ et X₁ sont tels que définis ci-dessus pour (II).

La préparation du composé de formule (V) est décrite dans les demandes de brevet EP-A-673928 et WO 97/32852.

Pour transformer le diester racémique de formule (V) en hémiester chiral de formule (VI), on effectue une hydrolyse enzymatique énantiosélective en choisissant des conditions semblables à celles décrites ci-dessus.

L'hémiester chiral de formule (VI) est isolé du milieu soit par extraction sélective, soit par précipitation après acidification de la phase aqueuse.

Le composé de formule (VI) est nouveau et constitue un aspect ultérieur de la présente invention.

Selon un aspect ultérieur, la présente invention est relative à l'utilisation d'un composé de formule (II) pour la préparation d'un composé de formule : 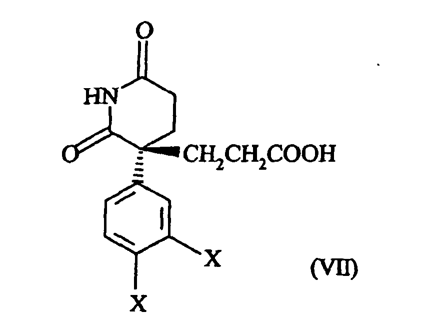 dans laquelle X est tel que défini ci-dessus ; l'hydrolyse d'un ester de formule (II) étant réalisée dans des conditions permettant de conserver la stéréochimie du carbone en 3 de la pipéridinedione. Ainsi, on peut utiliser l'action d'un acide, par exemple l'action d'un acide carboxylique en présence d'un acide minéral, de préférence l'acide acétique en présence d'acide chlorhydrique.

Ainsi, la présente invention concerne un procédé de préparation d'un composé de formule (VII) par hydrolyse d'un ester de formule (II).

Selon un autre aspect, la présente invention est relative à l'utilisation d'un composé de formule (II) pour la préparation d'un composé de formule :

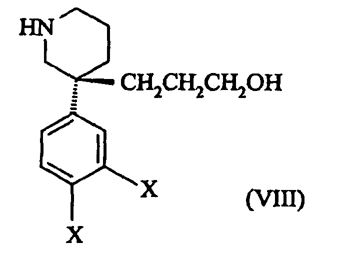

La réduction du composé de formule (II) en un composé de formule (VIII) peut être effectuée par action d'un agent réducteur.

Les agents de réduction utilisés sont des complexes du borane tels que par exemple le borane-tétrahydrofurane ou le borane-diméthylsulfure ou encore un hydrure mixte alcalin tel que l'hydrure de lithium aluminium ou l'hydrure de sodium bis (2-méthoxyéthoxy)aluminium en solution dans le toluène (Red-Al®). Ces réductions se font sans racémisation, l'agent de réduction préféré est le complexe borane-tétrahydrofurane.

La réduction avec le borane est conduite dans un solvant de préférence aprotique tel que le tétrahydrofurane à la température de reflux du solvant. En général, après 1 à 6 heures de chauffage, la réduction est complète et la pipéridine 3,3-disubstituée est isolée, selon les méthodes conventionnelles, en détruisant d'abord l'excès de borane avec du méthanol. La base libre peut être isolée par évaporation du solvant, puis on reprend le résidu par de l'eau, on acidifie avec de l'acide chlorhydrique, on traite avec une base, de préférence l'hydroxyde de sodium, et on extrait avec un solvant.

La base libre de formule (VIII) peut être transformée en un de ses sels selon les techniques bien connues. Le borane utilisé pour la réduction peut être généré *in situ* selon les méthodes classiques.

Ainsi, la présente invention concerne un procédé de préparation d'un composé de formule (VIII) par réduction d'un composé de formule (II).

Enfin, selon un autre aspect, la présente invention est relative à l'utilisation d'un composé de formule (VI) pour la préparation d'un composé de formule : 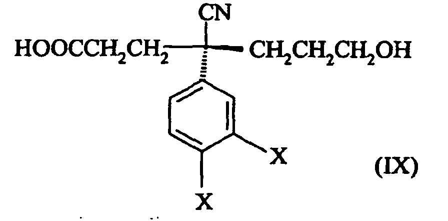 dans laquelle X est tel que défini ci-dessus pour (II), par réduction en présence d'un hydrure alcalin tel que, par exemple, LiAlH₄ ou LiAlH₃ dans le méthanol.

Par cyclisation du composé de formule (IX) dans les conditions décrites ci-dessus, on prépare un composé de formule :

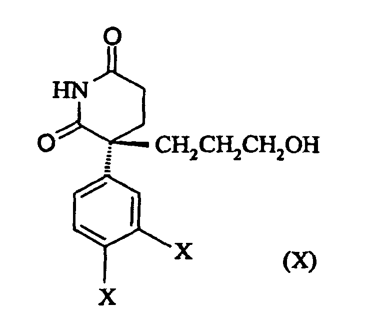

Le composé de formule (X) est décrit dans la demande internationale de brevet WO 98/05640.

Ainsi, la présente invention concerne un procédé de préparation d'un composé de formule (IX) par réduction d'un composé de formule (VI).

Dans la présente description, les abréviations suivantes sont utilisées :
DMSO : diméthylsulfoxyde
MTBE : méthyl-*tert*-butylether
THF : tétrahydrofurane
éther iso : éther isopropylique
TA : température ambiante
HPLC : chromatographie liquide haute pression
IR : infra rouge
RMN : résonance magnétique nucléaire à 250 ou 300 MHz
δ : déplacement chimique exprimé en p.p.m.
s : singulet ; d : doublet, d de d : doublet de doublet ; m : multiplet ou massif.

Les exemples suivants illustrent l'invention.

### EXEMPLE 1 :

Ester méthylique de l'acide 3-(3,4-dichlorophényl)-2,6-dioxopipéridine-3-propionique.

Dans un ballon de 500 ml, on place 33 g d'acide 3-(3,4-dichlorophényl)-2,6-dioxopipéridine-3-propionique dans 300 ml de méthanol et 1,5 g d'H₂SO₄ puis on chauffe à reflux pendant 45 minutes. Le méthanol est évaporé puis le résidu est repris par 300 ml d'éther et agité 2 heures à TA. Le précipité formé est filtré, rincé à l'éther iso puis séché sous vide à 40°C. On obtient 29,6 g de l'ester attendu. Rendement 86 %.
RMN (DMSO) (solvant δ ¹H : 2,5 ppm) :
δ:11(s,1H);7,26-7,65(m,3H);3,51(s,3H);2,06-2,51(m,8H)

### EXEMPLE 2 :

### Ester méthylique de l'acide 3-(3,4-dichlorophényl)-2,6-dioxopipéridine-3-propionique, isomère (+) (Procédé 1).

A 1 g d'ester méthylique de l'acide 3-(3,4-dichlorophényl)-2,6-dioxopipéridine-3-propionique, racémique dans 20 ml de MTBE (50 g/l) sont ajoutés 5 g de lipase de *Candida Cylindracea* L034 (Biocatalysts) en suspension dans 25 ml de tampon phosphate 0,1M pH7,0. Le mélange réactionnel est thermostaté à 40°C et mis sous agitation pendant 5 heures. L'avancement de la réaction est suivi par HPLC. Après 5 heures de réaction, 48 % du produit de départ est hydrolysé ; la réaction est arrêtée. 80 ml de MTBE sont ajoutés au mélange réactionnel et celui-ci est placé dans la glace, 90 ml d'acétone sont incorporés pour faire précipiter l'enzyme. Le précipité est filtré sur filtre de cellulose puis les solvants organiques sont évaporés. Dans la phase aqueuse restante sont ajoutés 2 équivalents de triéthylamine pour amener le pH du milieu à 8,5. L'ester non hydrolysé est extrait avec 3 x 25 ml de dichlorométhane. La phase dichlorométhane est séchée sur sulfate de magnésium anhydre. Après filtration et évaporation à sec (sous vide) du dichlorométhane, 500 mg d'une gomme jaune sont isolés correspondant au composé attendu (pureté 96 % par analyse HPLC). Une extraction à pH acide est ensuite réalisée. On ajoute dans la phase aqueuse, sous agitation, 25 ml de dichlorométhane et 2 ml H₂SO₄ 1N, puis on extrait deux fois avec 25 ml de dichlorométhane. On sèche la phase organique sur sulfate de magnésium anhydre, on filtre et on évapore sous vide à sec. On obtient 450 mg de solide blanc analysé en HPLC : 100 % d'acide 3-(3,4-dichlorophényl)-2,6-dioxopipéridine-3-propionique, isomère (-).
RMN (DMSO) (solvant δ ¹H : 2,5 ppm) :
Acide 3-(3,4-dichlorophényl)-2,6-dioxopipéridine-3-propionique,isomère (-) :
δ:12,10(s,1H);11,0(s,1H);7,66(d,1H);7,55(d,1H);7,28(dd,1H) ,2,50-2,40(m,2H);2,25-2,0(m,6H)
Composé attendu :
δ:11,0(s,1H);7,66(d,1H);7,55(d,1H);7,28(dd,1H),3,52(s,3H); 2,50-2,40(m,2H);2,35-2,0(m,6H)
Acide 3-(3,4-dichlorophényl)-2,6-dioxopipéridine-3-propionique,isomère (-). 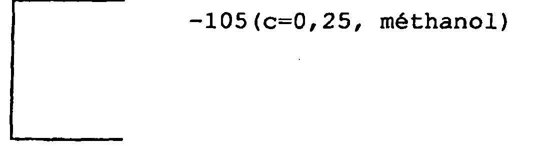 Composé attendu :

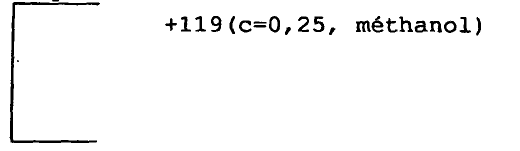

### EXEMPLE 3 :

### Ester méthylique de l'acide 3-(3,4-dichlorophényl)-2,6-dioxopipéridine-3-propionique, isomère (+) (Procédé 2) A)

Monométhylester de l'acide 4-cyano-4-(3,4-dichlorophényl)heptanedioique isomère (-).

A 12 g d'ester diméthylique de l'acide 4-cyano-4-(3,4-dichlorophényl) heptanedioique acide sont dissous dans 53 ml de MTBE et 1 g de lipase de *Candida Cylindracea* L034 (Biocatalysts) sont dissous dans 66 ml de tampon phosphate 50 mM pH7. Les deux solutions sont mélangées dans un ballon tricol de 500 ml. Le mélange réactionnel est agité vigoureusement de manière à créer une émulsion. La température est fixée à 20°C. La réaction est arrêtée après 5 heures. L'avancement de la réaction est suivie par HPLC.

La séparation du monométhylester est réalisée comme suit : 240 ml d'acétone sont ajoutés au mélange réactionnel et le milieu est placé à 5°C pendant 2 heures pour faire précipiter la lipase. Après 2 heures, le précipité est filtré sur filtre de cellulose. Les solvants organiques sont évaporés de la phase liquide sous pression réduite. La phase aqueuse résultante est basifiée à pH9 (NaHCO₃) et extraite au toluène (240 ml). La phase toluène est évaporée à sec. Il en résulte une poudre contenant 90 % d'ester diméthylique de départ et 10 % de monométhylester attendu. La phase aqueuse est acidifiée à pH 2,5 (HCl 1N) puis extraite au CH₂Cl₂ (240 ml). Après évaporation, le monométhylester attendu est isolé sous forme d'une poudre blanche : 7,8 g. Pureté : 99 %.

On peut également séparer le monométhylester de la façon suivante :

En fin de réaction, le mélange est basifié à pH = 9 par addition de soude puis on sépare par décantation la phase aqueuse et la phase organique. La phase organique est acidifiée jusqu'à pH = 2,5 par ajout d'HCl. Il se forme un précipité qui correspond à l'hémiester, celui-ci est récupéré par filtration.

L'hémiester peut être purifié par recristallisation dans l'acétonitrile.

| Analyse centésimale : | C | H | N |
|---|---|---|---|
| Théorique | 52,34 | 4,32 | 4,06 |
| Mesuré | 52,24 | 4,33 | 4,10 |

Point de fusion : 110,2°C.
IR(nujol) : 2238 cm⁻¹(nitrile);1740 cm⁻¹(ester);1693 cm⁻¹(acide carboxylique)
RMN(DMSO):δ
12,30(s,1H);7,73(d,1H);7,72(d,1H);7,46(dd,1H);3,51(s,3H); 2,40-2,28(m,4H);2,35(m,1H);2,25(m,1H);2,07(m,1H);1,95(m,1H)
RMN ¹³C (DMSO) :
δ : 173,3 ; 172,2 ; 138,3 ; 132,5 ; 131,7 ; 131,6 ; 128,6 ; 127,2 ; 121,1 ; 52,0 ; 47,0 ; 34,8 ; 34,7 ; 30,5 ; 30,3

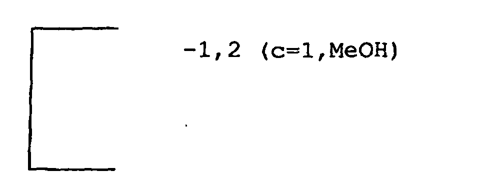

### B) Méthylester de l'acide 3-(3,4-dichlorophényl)-2,6-dioxopipéridine-3-propionique, (isomère +).

Ce composé peut être préparé par chauffage en présence d'anhydride de l'acide méthanesulfonique selon le procédé décrit ci-après.

Dans un tricol de 100 ml sous courant d'azote, sont introduits 1 g d'anhydride de l'acide méthanesulfonique et 60 mg d'acide méthanesulfonique (100 %). Le mélange est chauffé jusqu'à fusion. 1 g du composé de l'étape A est ajouté au milieu réactionnel et l'ensemble est porté à 100°C. La réaction est suivie par chromatographie sur couche mince (CH₂Cl₂ : MeOH, 95:5). Après une heure, la réaction est arrêtée. Le milieu est refroidi à température ambiante et l'anhydride est hydrolysé par ajout d'eau. Le produit de réaction est extrait au dichlorométhane et purifié par lavage avec une solution aqueuse de NaHCO₃ 1M. Après séchage sur MgSO₄ anhydre et évaporation sous vide du dichlorométhane, la glutarimide ester est isolé sous forme d'huile jaune. Le produit attendu cristallise lentement à l'air : obtient 0,85 g (analyse HPLC : pureté chimique 98 %). Point de fusion 105°C.

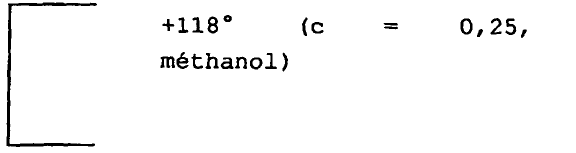

### EXEMPLE 4 :

### Fumarate de 3-(3,4-dichlorophényl)-3-(3-hydroxypropyl)pipéridine.

Sous azote, on met en solution 17,2 g d'ester méthylique de l'acide 3-(3,4-dichlorophényl)-2,6-dioxopipéridine-3-propionique dans 50 ml de THF. On ajoute en 10 minutes, à 10°C, 200 ml de borane 1M dans le THF. Après 2 heures de chauffage à reflux, on ajoute 60 ml supplémentaire de borane 1M dans le THF et on maintient le chauffage à reflux pendant 1 heure de plus. On détruit l'excès de borane par du méthanol. Après un dégazage important, on chauffe 30 minutes à reflux puis on évapore les solvants. Le résidu est repris par 300 ml d'eau et 10 g d'H₂SO₄ puis on chauffe 2 heures à reflux et on laisse une nuit à TA. On ajoute 25 ml de soude concentrée puis on extrait (2 fois) par 80 ml de butanol. La phase organique est lavée par 1000 ml d'eau puis concentrée et le résidu est repris par 100 ml d'isopropanol, on chauffe à reflux et on salifie par addition de 7 g d'acide fumarique dans 75 ml d'isopropanol et on laisse revenir à TA. Le précipité formé est filtré puis séché sous vide, on obtient 13,23 g du composé attendu.

Les filtrats sont concentrés et on isole 0,70 g supplémentaire du composé attendu. Rendement global : 81,5 %.

### EXEMPLE 5 :

### Acide 3-(3,4-dichlorophényl)-2,6-dioxopipéridine-3-propionique

### Méthylester de 3-(3,4-dichlorophényl)-2,6-dioxopipéridine-3-propionique, isomère (+).

Ce composé est préparé à l'EXEMPLE 3 étape C.

### B) Acide 3-(3,4-dichlorophényl)-2,6-dioxopipéridine-3-propionique.

Dans un tricol de 100 ml sont introduits 0.668 g du composé de l'étape précédente, 2 ml d'acide acétique et 0.10 ml d'acide chlorhydrique concentré. L'ensemble est porté à 70°C. Après 2 heures, le produit de réaction précipite et la réaction est arrêtée. Après retour à température ambiante, 2 ml d'eau sont rajoutés au milieu réactionnel. Le produit de réaction est filtré sur fritté, lavé à l'eau, puis recristallisé dans l'acide acétique. On obtient 0,47 g du composé attendu. (Rendement 70 %).

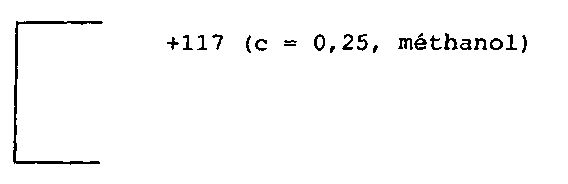

### EXEMPLE 6

### Acide 4-cyano-4-(3,4-dichlorophényl)-7-hydroxy heptanoique.

Sous un courant d'azote, sont introduits 231 mg de LiBH₄ et 30 ml de MTBE puis 429 µl de méthanol dilué dans 30 ml de MTBE sont ajoutés gouttes à gouttes. 1 g de composé préparé à l'EXEMPLE 3, étape A en solution dans 80 ml de MTBE éther est ajouté au milieu réactionnel et l'ensemble est chauffé à reflux. Après trois heures, le milieu réactionnel est placé dans la glace et une solution de HCl 1N est ajoutée. Lorsqu'il n'y a plus de dégagement gazeux, le produit de réaction est extrait au dichlorométhane. Après séchage sur sulfate de magnésium anhydre et évaporation du solvant sous vide, le produit est isolé sous forme d'une gomme blanche.

Le produit est recristallisé dans 6 ml de toluène et on obtient 600 mg d'un composé attendu sous forme d'une poudre blanche. 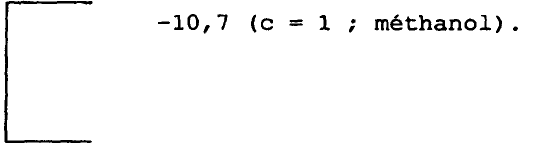 RMN (DMSO) (solvant δ ¹H : 2,5 ppm) :
δ : 12,30 (s1, 1H) ; 7,69 (d, 1H) ; 7,65 (d, 1H) ; 7,42 (dd, 1H) ; 4,5 (s1, 1H) ; 3,33 (t, 2H) ; 2,35-2,2 (m, 3H) ; 2,1-1,8 (m, 3H) ; 1,50-1,10 (m, 2H).

## Revendications

1. Un composé de configuration S répondant à la formule : 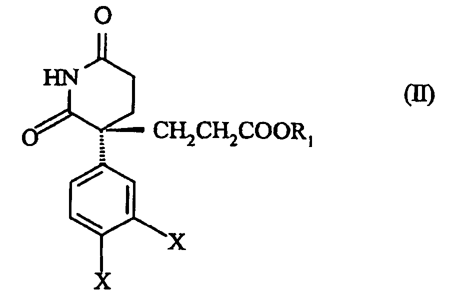 dans laquelle :
- X représente un halogène ;
- R₁ représente un alkyle linéaire en C₁-C₄.

2. Un composé selon la revendication 1 de formule (II) dans laquelle X représente un atome de chlore ou un atome de fluor.

3. Un composé selon la revendication 1 de formule (II) dans laquelle X = Cl et R₁ = CH₃.

4. Un procédé de préparation d'un composé selon la revendication 1 **caractérisé en ce que** l'on effectue une hydrolyse enzymatique énantiosélective du composé de formule : 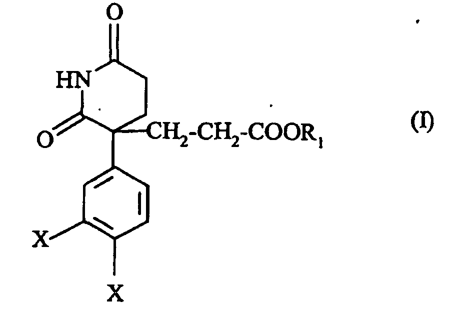 dans laquelle X représente un halogène et R₁ représente un alkyle linéaire en C₁-C₄

5. Un procédé selon la revendication 4 **caractérisé en ce que** on utilise une enzyme choisie parmi les lipases, les estérases et les protéases.

6. Un procédé selon la revendication 5 **caractérisé en ce qu'**on utilise la lipase ou l'estérase de *Candida Cylindracea* ou de *Candida Rugosa,* séparément ou en mélange.

7. Un procédé pour la préparation d'un composé selon la revendication 1, **caractérisé en ce qu'**il consiste à effectuer une cyclisation du composé de formule : 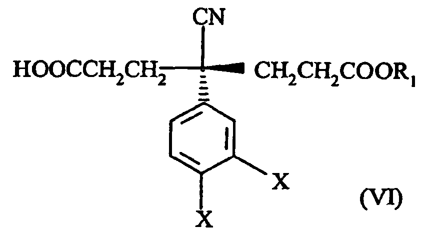 dans laquelle R₁ et X sont tels que définis pour (II) dans la revendication 1.

8. Procédé selon la revendication 7 **caractérisé en ce que** la cyclisation est effectuée en présence d'un catalyseur entre 20°C et 130°C.

9. Procédé selon la revendication 8 **caractérisé en ce que** la cyclisation est réalisée en présence d'un anhydride d'acide ou d'un acide, ou d'un mélange d'un anhydride d'acide et d'un acide.

10. Procédé selon la revendication 9 **caractérisée en ce que** la cyclisation est réalisée en présence d'anhydride méthanesulfonique et d'acide méthanesulfonique ou en présence d'anhydride triflique et d'acide triflique.

11. Un procédé pour la préparation d'un composé de formule : 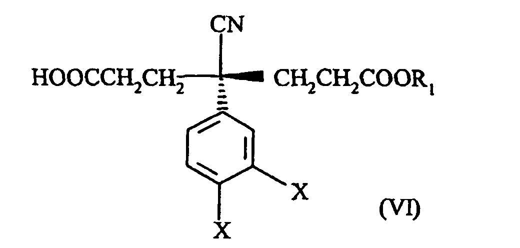 dans laquelle R₁ et X sont tels que définis pour (II) dans la revendication 1, **caractérisé en ce qu'**il consiste à traiter par une enzyme un composé de formule :

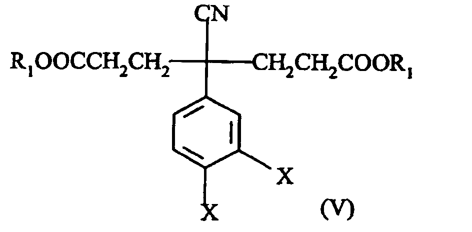

12. Procédé selon la revendication 11 **caractérisé en ce que** l'on utilise une enzyme choisie parmi les lipases, les estérases et les protéases.

13. Procédé selon la revendication 12 **caractérisé en ce que** à l'on utilise la lipase ou l'estérase de *Candida Cylindracea* ou de *Candida Rugosa,* séparément ou en mélange.

14. Un composé de formule : 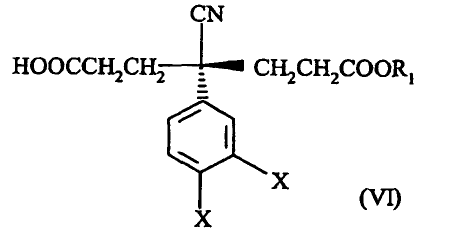 dans laquelle R₁ et X sont tels que définis pour (II) dans la revendication 1.

15. Utilisation d'un composé de formule (II) selon la revendication 1 pour la préparation d'un composé de formule : 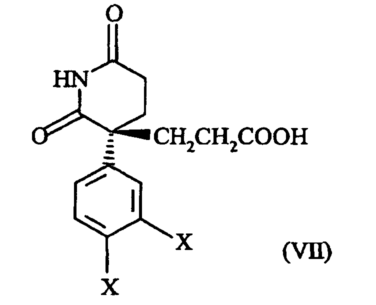 dans laquelle X représente un halogène, par hydrolyse.

16. Utilisation d'un composé de formule (II) selon la revendication 1 pour la préparation d'un composé de formule : 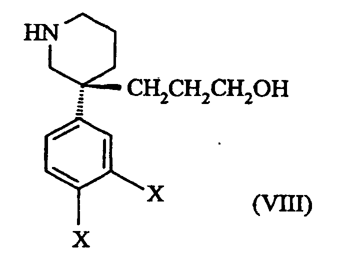 dans laquelle X représente un halogène, par réduction en présence d'un agent réducteur.

17. Utilisation d'un composé de formule (VI) selon la revendication 14 pour la préparation d'un composé de formule : 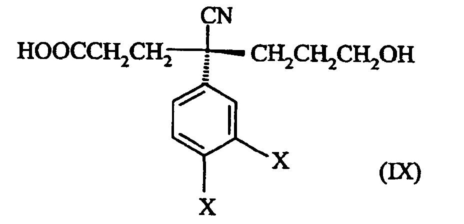 dans laquelle X représente un halogène, par réduction en présence d'un hydrure alcalin.

## Patentansprüche

1. Verbindung der Konfiguration S, entsprechend der Formel (II) 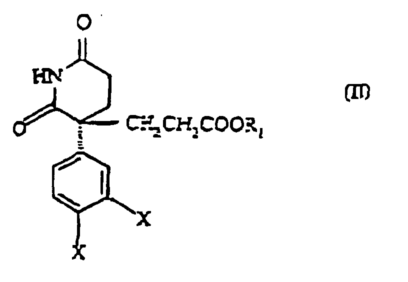 in der:
- X ein Halogen darstellt;
- R₁ ein lineares Alkyl mit 1 bis 4 Kohlenstoffatomen darstellt.

2. Verbindung nach Anspruch 1 der Formel (II), in der X ein Chloratom oder ein Fluoratom darstellt.

3. Verbindung nach Anspruch 1 der Formel (II), in der
X = Cl und R₁ = CH₃ sind.

4. Verfahren zur Herstellung einer Verbindung nach Anspruch 1, **dadurch gekennzeichnet, daß** man eine enantioselektive enzymatische Hydrolyse der Verbindung der Formel (I) 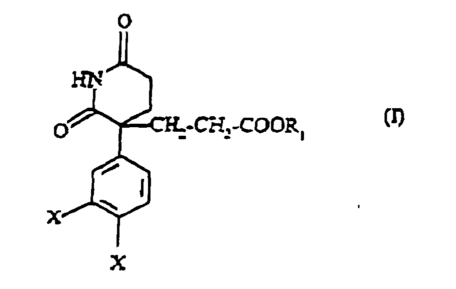 durchführt,
in der X ein Halogen darstellt und R₁ ein lineares Alkyl mit 1 bis 4 Kohlenstoffatomen darstellt.

5. Verfahren nach Anspruch 4, **dadurch gekennzeichnet, daß** man ein Enzym verwendet, ausgewählt unter den Lipasen, den Esterasen und den Proteasen.

6. Verfahren nach Anspruch 5, **dadurch gekennzeichnet, daß** man die Lipase oder Esterase von *Candida Cylindracea* oder von *Candida Rugosa* verwendet, getrennt oder in Mischung.

7. Verfahren zur Herstellung einer Verbindung nach Anspruch 1, **dadurch gekennzeichnet, daß** es darin besteht, eine Cyclisierung der Verbindung der Formel (VI) 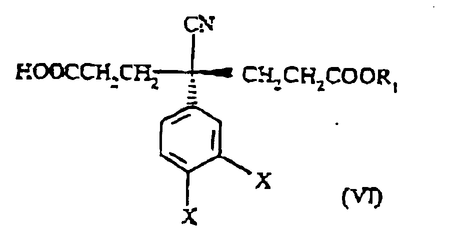 durchzuführen, in der R₁ und X wie bei (II) in Anspruch definiert sind.

8. Verfahren nach Anspruch 7, **dadurch gekennzeichnet, daß** die Cyclisierung in Anwesenheit eines Katalysators zwischen 20 °C und 130 °C durchgeführt wird.

9. Verfahren nach Anspruch 8, **dadurch gekennzeichnet, daß** die Cyclisierung in Anwesenheit eines Säureanhydrides oder einer Säure oder einer Mischung von einem Säureanhydrid und einer Säure durchgeführt wird.

10. Verfahren nach Anspruch 9, **dadurch gekennzeichnet, daß** die Cyclisierung in Anwesenheit von Methansulfonsäure-anhydrid und Methansulfonsäure oder in Anwesenheit von Triflinsäure-anhydrid und Triflinsäure durchgeführt wird.

11. Verfahren zur Herstellung einer Verbindung der Formel (VI) 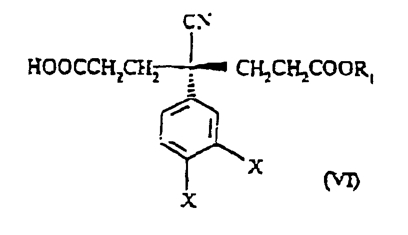 in der
R₁ und X wie bei (II) in Anspruch 1 definiert sind, **dadurch gekennzeichnet, daß** es darin besteht, eine Verbindung der Formel (V) 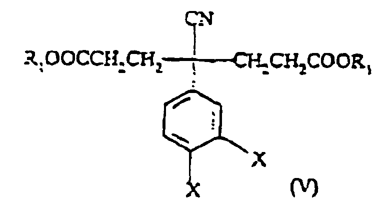 mit einem Enzym zu behandeln.

12. Verfahren nach Anspruch 11, **dadurch gekennzeichnet, daß** man ein Enzym verwendet, ausgewählt unter den Lipasen, den Esterasen und den Proteasen.

13. Verfahren nach Anspruch 12, **dadurch gekennzeichnet, daß** man die Lipase oder Esterase von *Candida Cylindracea* oder von *Candida Rugosa* verwendet, getrennt oder in Mischung.

14. Verbindung der Formel (VI) 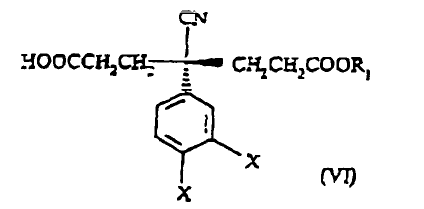 in der
R₁ und X wie bei (II) in Anspruch 1 definiert sind.

15. Verwendung einer Verbindung der Formel (II) nach Anspruch 1 für die Herstellung einer Verbindung der Formel (VII) 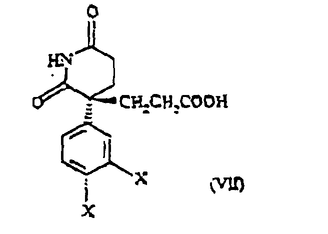 in der X ein Halogen darstellt, durch Hydrolyse.

16. Verwendung einer Verbindung der Formel (II) nach Anspruch 1 für die Herstellung einer Verbindung der Formel (VIII) 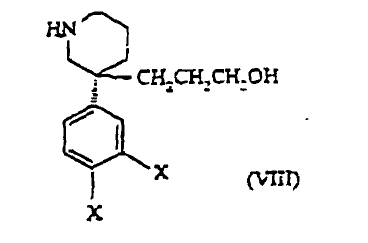 in der X ein Halogen darstellt, durch Reduktion in Anwesenheit eines Reduktionsmittels.

17. Verwendung einer Verbindung der Formel (VI) nach Anspruch 14 für die Herstellung einer Verbindung der Formel (IX) 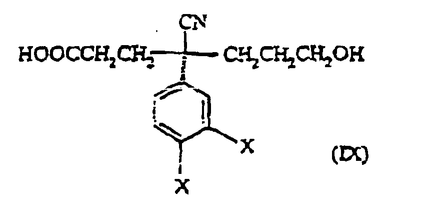 in der X ein Halogen darstellt, durch Reduktion in Anwesenheit eines Alkalihydrides.

## Claims

1. Compound of S configuration corresponding to the formula: 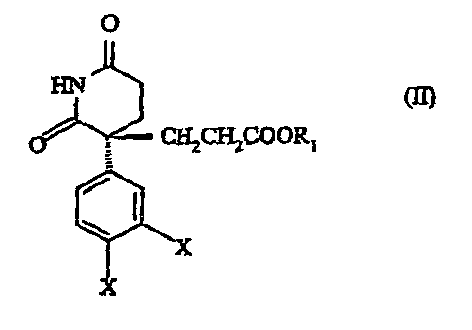 in which:
- X represents a halogen,
- R₁ represents a linear C₁-C₄ alkyl.

2. Compound according to Claim 1 of formula (II), in which X represents a chlorine atom or a fluorine atom.

3. Compound according to Claim 1 of formula (II), in which X = Cl and R₁ = CH₃.

4. Process for the preparation of a compound according to Claim 1, **characterized in that** an enantioselective enzymatic hydrolysis of the compound of formula: 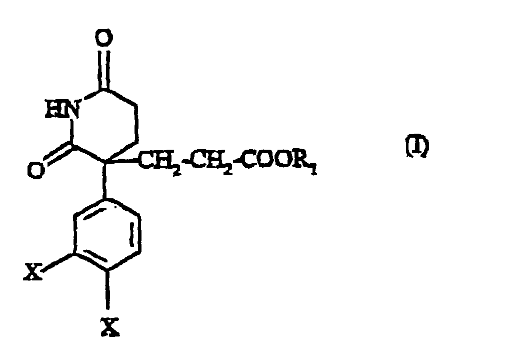 in which X represents a halogen and R₁ represents a linear C₁-C₄ alkyl,
is carried out.

5. Process according to Claim 4, **characterized in that** use is made of an enzyme chosen from lipases, esterases and proteases.

6. Process according to Claim 5, **characterized in that** use is made of *Candida cylindracea* or *Candida rugosa* lipase or esterase, separately or as a mixture.

7. Process for the preparation of a compound according to Claim 1, **characterized in that** it consists in carrying out a cyclization of the compound of formula: 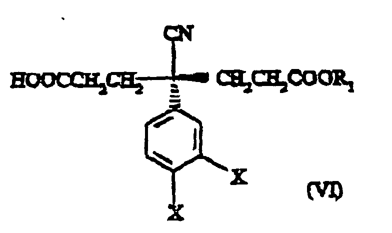 in which R₁ and X are as defined for (II) in Claim 1.

8. Process according to Claim 7, **characterized in that** the cyclization is carried out in the presence of a catalyst between 20°C and 130°C.

9. Process according to Claim 8, **characterized in that** the cyclization is carried out in the presence of an acid anhydride or of an acid, or of a mixture of an acid anhydride and of an acid.

10. Process according to Claim 9, **characterized in that** the cyclization is carried out in the presence of methanesulphonic anhydride and of methanesulphonic acid or in the presence of triflic anhydride and of triflic acid.

11. Process for the preparation of a compound of formula: 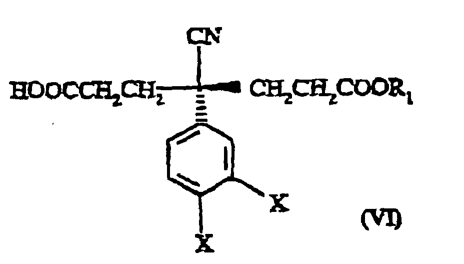 in which R₁ and X are as defined for (II) in Claim 1, **characterized in that** it consists in treating, with an enzyme, a compound of formula:

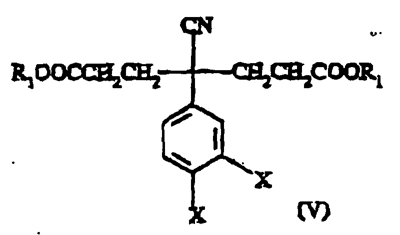

12. Process according to Claim 11, **characterized in that** use is made of an enzyme chosen from lipases, esterases and proteases.

13. Process according to Claim 12, **characterized in that** use is made of *Candida* *cylindracea* or *Candida rugosa* lipase or esterase, separately or as a mixture.

14. Compound of formula: 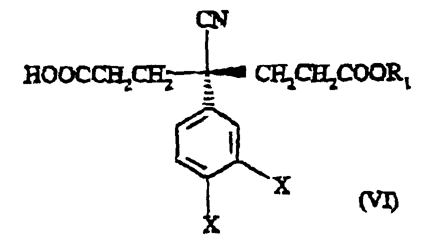 in which R₁ and X are as defined for (II) in Claim 1.

15. Use of a compound of formula (II) according to Claim 1 in the preparation of a compound of formula: 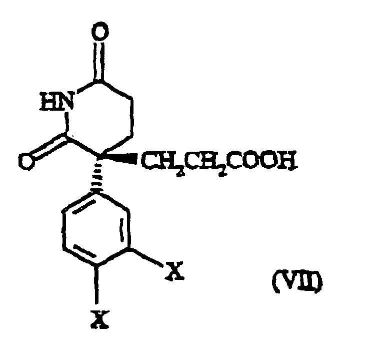 in which X represents a halogen, by hydrolysis.

16. Use of a compound of formula (II) according to Claim 1: in the preparation of a compound of formula: 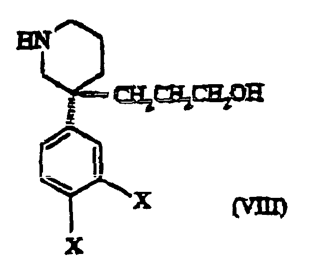 in which X represents a halogen, by reduction in the presence of a reducing agent.

17. Use of a compound of formula (VI) according to Claim 14 in the preparation of a compound of formula: 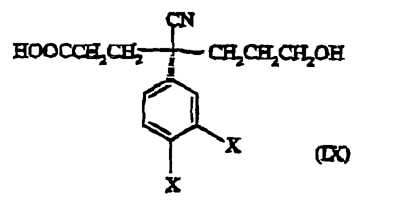 in which X represents a halogen, by reduction in the presence of an alkaline hydride.
